# DEMANDE DE BREVET EUROPEEN

(11) **EP 0 769 302 A1**
(43) Date de publication de la demande: **23.04.1997**
(21) Numéro de dépôt: 96402214.9
(22) Date de dépôt: 16.10.1996
(51) Int. Cl.: A61M 5/32

(54) **Dispositif de protection d'une aiguille hypodermique**

(30) Priorité: 20.10.1995 FR 9512602
(71) Demandeur: B. BRAUN CELSA (société anonyme), F-86360 Chasseneuil du Poitou (FR)
(72) Inventeur: Camus, M. Michel, 59175 Templemars (FR)
(74) Mandataire: Lerner, François

(57) **Abrégé**

L'invention se rapporte à un instrument 1 comprenant une aiguille 2 hypodermique et un dispositif 6 de protection qui est constitué par deux éléments 7 symétriques plats, mobiles par rapport au corps 3 de l'instrument autour d'axes parallèles à une direction sécante à l'axe longitudinal de l'aiguille 2, l'instrument étant remarquable en ce que :
- les éléments 7 symétriques plats sont constitués chacun par une lame 7 souple, de souplesse déterminée,
- avec ces éléments 7 coopère une pièce 9 mobile qui, lorsqu'elle est sollicitée vers une surface 5R réceptrice de l'instrument, et ce, cependant que ladite aiguille 2 en est extraite, assure l'application progressive de chaque lame 7 contre cette aiguille 2, ladite application ayant lieu au fur et à mesure de l'extraction.

## Description

L'invention concerne un dispositif de protection d'une aiguille hypodermique sur un instrument comportant une telle aiguille.

L'invention s'applique aux instruments qui comprennent une aiguille tubulaire ayant une partie distale perforante et une partie proximale destinée à être raccordée à un conduit de circulation d'un liquide.

L'invention s'applique indifféremment aux instruments d'injection d'un liquide dans un milieu de réception, tel dans un site implanté, ou, aux instruments de prélèvement d'un liquide dans un milieu de prélèvement.

Le terme "site" désigne ci-après tout milieu dans lequel l'aiguille peut être engagée.

Le dispositif de l'invention est destiné à la prévention de piqûres accidentelles de personnes qui manipulent ces instruments.

De tels accidents surviennent essentiellement lors du retrait d'une aiguille d'un milieu d'injection ou de prélèvement.

Pour, dans le domaine des instruments équipés d'une aiguille hypodermique, réduire la probabilité de piqûres, on connaît de très nombreux dispositifs mettant en oeuvre au moins un élément de protection qui, constitué en matériau rigide et au moins indirectement associé à l'extrémité proximale de l'aiguille, d'une part, est mobile entre deux positions, dont une première position dans laquelle il dégage largement l'aiguille de manière à permettre son utilisation et une seconde position dans laquelle il coiffe au moins l'extrémité distale de cette aiguille.

US-A-4 888 001 est relatif à un instrument à aiguille hypodermique qui comprend un tel dispositif de protection.

Ce dispositif a ses avantages ; mais le demandeur s'est attaché à améliorer les caractéristiques des dispositifs de protection d'aiguilles hypodermiques existants en cherchant une solution encore plus appropriée au problème ci-dessous :
- sécurité pour l'utilisation et pour le patient,
- prix de revient maîtrisé,
- possibilité pour l'utilisateur de contrôler l'implantation et/ou l'extraction de l'aiguille,
- limiter l'encombrement du dispositif,
- assurer une manoeuvre aisée et sûre du dispositif,
- possibilité d'implantation de longue durée (au moins plusieurs heures).

En tant que solution générale en particulier aux questions de sécurité et de manoeuvre aisée et sûre, une première caractéristique de l'invention prévoit que le dispositif comprenne :
- une aiguille ayant une portion adaptée pour être insérée dans un corps, ladite portion ayant une longueur suivant un axe, ainsi qu'une extrémité distale effilée et une extrémité proximale,
- un support d'aiguille disposé autour d'un segment de l'aiguille vers l'extrémité proximale de celle-ci,
- un fourreau de protection de ladite portion d'aiguille comprenant au moins deux lames plates d'une façon générale ayant des extrémités proximale et distale et réalisées en un matériau flexible,
- au moins deux zones d'articulation, chaque zone d'articulation reliant l'extrémité proximale de chaque lame au support d'aiguille,
- un élément mobile présentant à travers lui une ouverture axiale au moins partiellement délimitée par des surfaces plates d'une façon générale, de manière à être disposé autour des lames, le long de l'axe de ladite portion d'aiguille, entre des positions haute et basse, dans lesquelles, en position haute, l'élément mobile est au-dessus des zones d'articulation, et, en position basse, l'élément mobile est sensiblement à l'extrémité distale des lames et ses dites surfaces plates d'une façon générale coopèrent avec une surface extérieure des lames pour provoquer un mouvement de pivotement vers le bas desdites lames en les rapprochant de ladite portion d'aiguille le long de son axe, lorsque l'élément mobile est déplacé de sa position haute vers sa position basse.

A noter notamment que la présence des lames flexibles essentiellement plates favorise également la limitation de l'encombrement, ainsi que l'implantation de longue durée, ces lames une fois appliquées le long de la surface (en particulier de la peau) à travers laquelle l'aiguille est implantée pouvant en particulier servir à un maintien efficace et peu gênant du dispositif sur le corps du patient.

En considérant plus particulièrement la question du suivi par l'utilisateur de l'implantation et/ou de l'extraction de l'aiguille, une caractéristique complémentaire de l'invention prévoit que chaque lame présente de préférence une surface intérieure opposée à ladite surface extérieure et qui est dépourvue de toute gorge propre à contenir ladite portion d'aiguille, de sorte que dans la position basse de l'élément mobile, ladite portion d'aiguille demeure apparente entre les lames et n'est pas enfermée à l'intérieur du fourreau de protection.

En relation avec la possibilité d'implantation de longue durée, une autre caractéristique de l'invention conseille que le segment d'aiguille autour duquel le support d'aiguille est disposé soit coudé.

A nouveau en relation avec la question de la sécurité, mais également avec celle de l'encombrement réduit, d'une manoeuvre aisée et sûre, une autre caractéristique de l'invention conseille que l'élément mobile comprenne des butées déviatrices qui sont disposées sous lesdites surfaces plates d'une façon générale de l'élément mobile et qui sont appliquées contre la surface extérieure des lames pour coopérer avec elles lorsque l'élément mobile est déplacé entre ses positions haute et basse.

Dans le cadre des mêmes considérations que ci-dessus, avec de surcroît une solution complémentaire au problème de l'implantation longue durée, une autre caractéristique de l'invention conseille que l'élément mobile se présente comme un cadre plat d'une façon générale suivant un plan général sensiblement perpendiculaire à l'axe de ladite portion d'aiguille .

A noter que cette caractéristique permet d'augmenter la stabilité du dispositif pendant ou après l'implantation de l'aiguille, sans former de surépaisseur importante, par trop gênante, au-dessus de la zone d'implantation, une fois l'aiguille implantée.

Toujours essentiellement pour les mêmes considérations, une autre caractéristique de l'invention conseille encore que :
- les lames flexibles présentent une largeur et une épaisseur,
- l'élément mobile présente deux patins latéraux s'étendant sensiblement perpendiculairement à l'axe de la portion d'aiguille et au plan général de chaque lame alors que l'élément mobile est dans sa position basse,
- la distance entre les patins est supérieure à la largeur des lames, et
- les patins présentent une épaisseur qui est supérieure à l'épaisseur des lames,
de sorte que lesdites lames glissent entre les patins lorsque l'élément mobile est déplacé entre ses positions haute et basse.

A nouveau en relation avec des questions de sécurité et de manoeuvre tant aisée que sûre, une caractéristique complémentaire de l'invention conseille que, sur leur surface extérieure, les lames présentent des butées saillantes avant et arrière disposées vers l'extrémité distale desdites lames, et ces butées présentent entre elles une distance adaptée pour verrouiller de manière réversible entre elles une distance adaptée pour verrouiller de manière réversible entre elles l'élément mobile dans la position basse de celui-ci.

En relation avec les mêmes questions, et pour compléter les dispositions relatives à une possible implantation de longue durée, une autre caractéristique de l'invention conseille que :
- le support d'aiguille présente au moins une rainure s'étendant perpendiculairement à l'axe de ladite portion d'aiguille,
- la rainure présente une longueur le long de son axe ainsi qu'une section supérieure longitudinalement délimitée par des rebords latéraux, et
- le dispositif comprend en outre une poignée amovible ayant des extrémités proximale et distale, ladite extrémité distale de la poignée étant engagée dans la rainure, sous lesdits rebords latéraux.

De préférence, la poignée amovible en question présentera deux mâchoires distales articulées d'une seule pièce avec deux branches de commande, les mâchoires étant engagées dans ladite rainure du support d'aiguille lorsque la poignée y est disposée.

En relation avec ce qui précède, et pour compléter la solution proposée aux questions de sécurité, de prix de revient maîtrisé, d'encombrement limité et de manoeuvre aisée et sûre, une autre caractéristique de l'invention conseille encore que le support d'aiguille comprenne en outre une plaque de butée disposée à l'intérieur de ladite rainure, sous la poignée amovible lorsque celle-ci y est disposée, la plaque de butée ayant des dimensions adaptées pour interagir en butée avec l'élément mobile lorsque celui-ci est dans sa position haute.

L'invention sera encore mieux comprise à l'aide de la description ci-après faite à titre d'exemple non limitatif, en regard du dessin ci-annexé qui représente:
- figures 1 à 3 : trois vues en coupe partielle selon un plan perpendiculaire à une surface réceptrice contre laquelle il est utilisé, d'un instrument équipé du dispositif selon l'invention dans différentes configurations comprises entre une situation implantée de l'aiguille et une situation de protection par les éléments à cet effet,

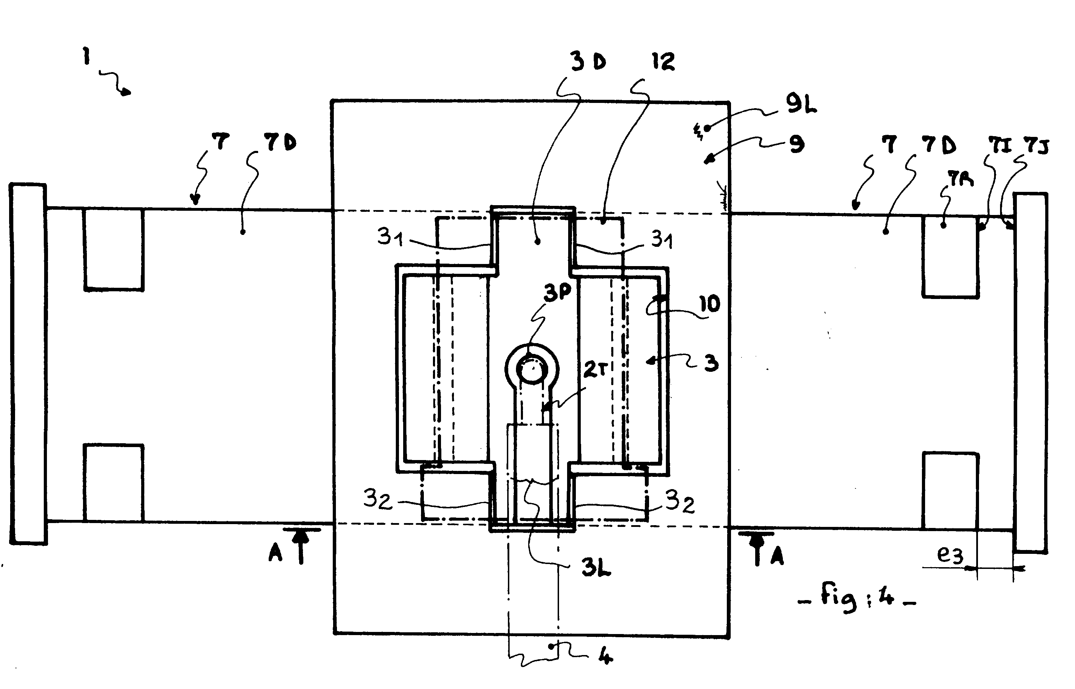

- figure 4 : une vue de dessus d'un instrument équipé du dispositif selon l'invention,
- figure 5 : une vue de dessous d'un instrument équipé du dispositif selon l'invention,

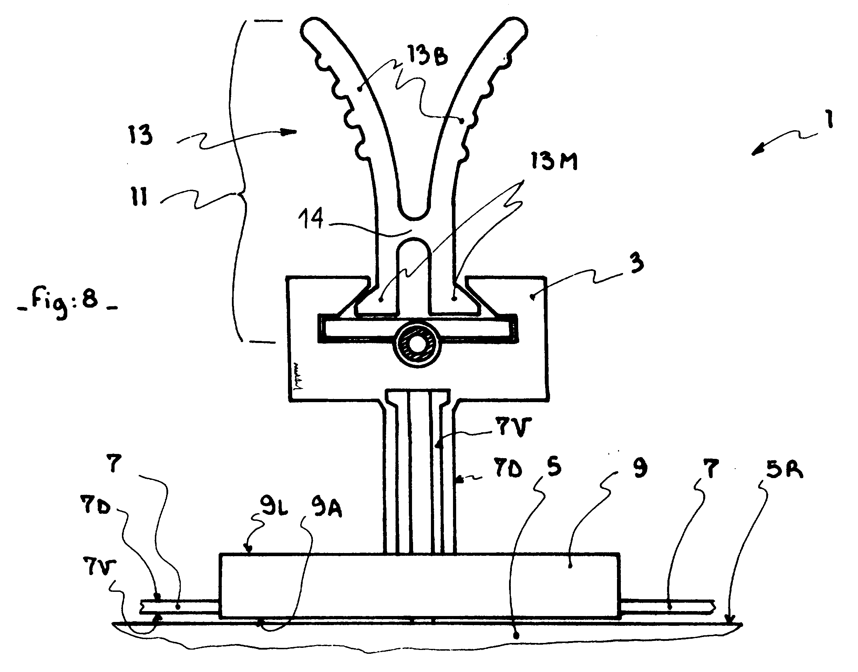

- figures 6 et 7 : deux vues en coupe selon A-A de l'instrument de la figure 4,
- figure 8 : une vue latérale de l'organe de préhension de l'instrument selon l'invention.

En se reportant aux dessins, on voit un instrument 1 qui met en oeuvre une aiguille 2 hypodermique comportant une partie distale 2D perforante (ou effilée) et une partie proximale 2P qui, portée par le corps 3 de l'instrument 1 (formant support pour l'aiguille), est au moins indirectement raccordée à une conduite 4 de circulation d'un liquide (non représentée).

Par exemple, l'instrument 1 est un instrument d'injection d'un liquide dans un milieu 5 d'injection situé sous une surface 5R, dite réceptrice.

A titre d'exemple, le milieu 5 consiste en un site implanté 5 sous une surface 5R réceptrice, telle la peau d'un patient (non représenté).

Avantageusement, mais non limitativement, l'extrémité 2D distale de l'aiguille 2 présente un biseau dit de "HUBER".

Tel que cela apparaît au dessin, la portion de l'aiguille 2 qui doit être implantée dans le corps 5, est approximativement orthogonale à la surface 5R réceptrice dans laquelle elle est engagée ; mais cela n'est pas limitatif.

A un moment choisi de l'utilisation de l'instrument 1, l'aiguille hypodermique doit être retirée du milieu 5 dans lequel elle est implantée.

Cette extraction est effectuée par action manuelle sur l'instrument 1 hypodermique dont le corps 3 présente à cet effet au moins une surface 3S, 3D, 12B de préhension au moins indirecte.

L'instrument 1 comprend, solidaire du corps 3 sur lequel l'aiguille 2 est retenue par son extrémité 2P proximale de manière à s'étendre sensiblement orthogonalement à l'une des faces 3V, dite ventrale, de ce corps 3, un dispositif (ou fourreau) 6 de protection qui est constitué par :
- deux éléments 7 symétriques plats d'une façon générale, qui sont mobiles par rapport audit support d'aiguille 3 autour d'axes 7A sensiblement parallèles à une direction sécante à l'axe longitudinal 2L de l'aiguille 2, et ce, de manière à pouvoir être placés dans au moins deux configurations fonctionnellement distinctes dont,
   - une configuration dite de protection, dans laquelle ils présentent chacun une face 7V, dite ventrale, qui s'étend au long de l'aiguille 2 et,
   - une configuration, dite d'utilisation, dans laquelle ils présentent chacun leur face 7V ventrale notablement écartée de l'aiguille 2,
- un moyen 8 de verrouillage de ces éléments 7 en configuration de protection.

Pour l'articulation en pivotement des lames (ou ailes) 7, deux charnières 7₁,7₂ sont prévues, chacune reliant l'extrémité proximale de l'aile 7 concernée au support d'aiguille 3. Il s'agit ici de deux lamelles amincies constituant deux "charnières intégrées" liant en une seule pièce plastique les lames et le support 3.

Tel que cela apparaît aux dessins, chaque lame 7 plate comprend deux faces 7V, 7D opposées dont, d'une part, une face dite ventrale 7V, destinée à venir en appui sur une surface 5R réceptrice, telle la peau, dans laquelle l'aiguille 2 hypodermique est engagée et, d'autre part, une face 7D dite dorsale, sur laquelle peut être exercée une action d'application de l'instrument 1 contre ladite surface 5R réceptrice, voire, une action d'application desdites ailes 7 contre l'aiguille 2 hypodermique.

La surface intérieure est exclusivement plate.

De manière remarquable :
- les éléments 7 symétriques plats sont constitués chacun par une lame 7 souple, de souplesse déterminée,
- avec ces éléments 7 coopère au moins une pièce 9 mobile qui, lorsqu'elle est sollicitée vers la surface 5R réceptrice, et ce pendant que ladite aiguille 2 en est extraite, assure l'application progressive de chaque lame 7 sensiblement contre cette aiguille 2, c'est-à-dire l'application vers ladite aiguille 2 de toute portion de lame 7 souple qui s'étend entre le corps 3 de l'instrument 1 et la surface 5R réceptrice dont l'aiguille 2 est extraite, ladite application ayant lieu au fur et à mesure de l'extraction.

En se reportant aux dessins, on constate que l'application a lieu de l'extrémité proximale 2P de l'aiguille 2 vers son extrémité distale 2D.

La sécurité d'utilisation d'un instrument équipé du dispositif selon l'invention est donc particulièrement accrue.

Les lames 7 ont chacune une dimension longitudinale supérieure à la longueur L de l'aiguille 2.

De manière remarquable, la pièce 9 mobile comprend au moins deux butées 9D qui, dites déviatrices, sont disposées pour s'appuyer chacune au moins localement contre la face dorsale 7D d'une lame 7 et, lors de l'extraction de l'aiguille 2 du milieu 5, autoriser le glissement de ladite surface 7D dorsale et induire, à la manière d'une poulie, la courbure et la déviation de la lame 7, et ce entre, d'une part le plan d'appui qu'elle trouve contre la surface 5R réceptrice et, d'autre part une direction sensiblement parallèle à la direction d'extraction de l'aiguille 2.

Les butées 9D sont "monoblocs" avec le support 3 et sont situées juste sous les surfaces plates 3₁,3₂ de ce support qui délimitent localement l'ouverture 10 ménagée à travers ledit support pour le passage des lames 7.

Avantageusement, mais non limitativement, de telles butées 9D sont constituées par des faces arrondies.

La présence de l'élément mobile 9 accroît encore la sécurité d'utilisation de l'instrument équipé du dispositif 6 de l'invention.

Chaque lame 7 est constituée dans un matériau de nature et d'épaisseur e₁ telles que :
- sa souplesse autorise son glissement contre une butée déviatrice 9D, avec déviation au moins à angle droit, sans détérioration de la lame 7,
- nonobstant cette souplesse, l'épaisseur de la lame 7 est au moins suffisante pour résister au déchirement dû à une application latérale accentuée contre l'extrémité 2D distale de l'aiguille 2.

L'homme du métier est à même de choisir le matériau et l'épaisseur convenables.

On notera que, de manière surprenante, le fourreau de protection que constituent les lames 7 rapprochées autour de l'aiguille est constitué avec un matériau souple.

De manière notable, la pièce 9 mobile présente une forme générale plate suivant un plan général 9' sensiblement perpendiculaire à l'axe 2L de l'aiguille. Elle comprend en outre, dans sa face d'appui contre la surface réceptrice 5R, des évidements 9E en vue du logement desdites lames 7 selon leur épaisseur e₁, lorsque l'instrument 1 est en configuration dite d'utilisation.

Les évidements 9E sont notamment délimités par une face 9M sensiblement parallèle à la face 9A d'appui sur la surface 5R réceptrice.

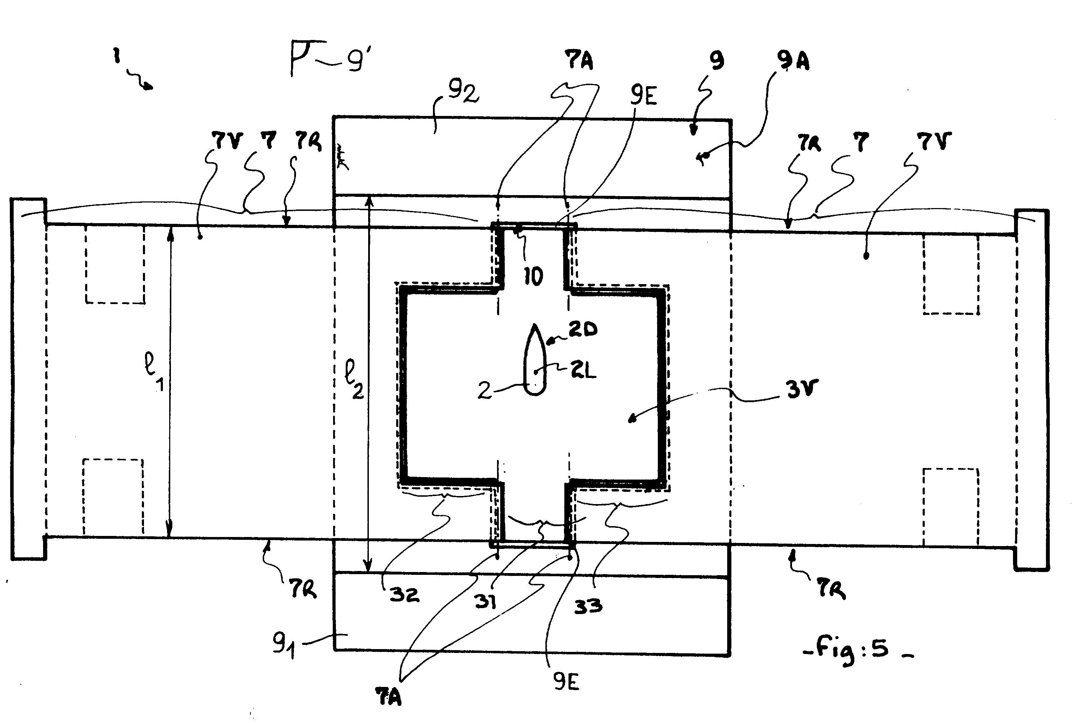

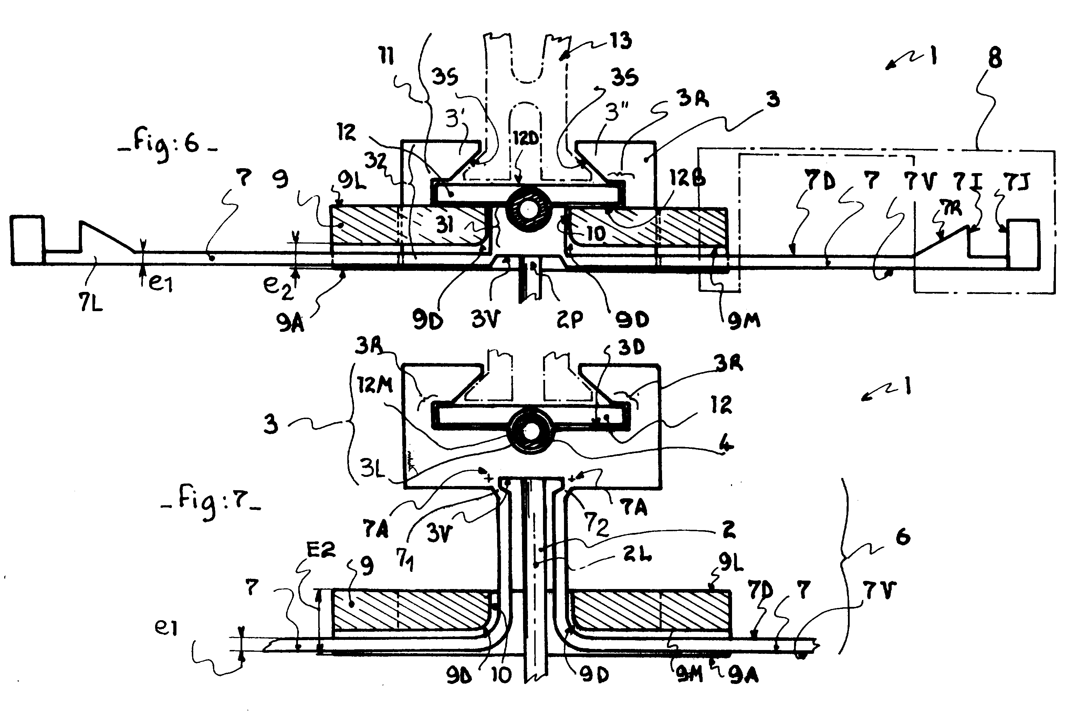

Plus précisément, l'élément mobile 9 se présente ici comme un cadre essentiellement plat et rectangulaire comprenant (sur sa surface inférieure) deux patins latéraux saillants d'appui 9₁,9₂ (figure 5) s'étendant perpendiculairement à l'axe 2L. La largeur l₂ entre les patins est supérieure à celle (l₁) de chaque lame, de même pour l'épaisseur (e₂ et e₁ ,respectivement; figure 6).

Par cela, la pièce 9 mobile conserve un appui sur la surface 5R réceptrice lors de l'extraction de l'aiguille 2, alors que les lames glissent entre les patins.

Pour effectuer l'extraction de l'aiguille hypodermique, il suffit de maintenir, notamment par au moins une action T, la pièce 9 mobile en appui contre la surface 5R réceptrice cependant qu'on exerce au moins une action T de traction sur le corps 3 de l'instrument 1.

Les lames 7 glissent contre la surface 5R réceptrice et contre les butées déviatrices 9D pour, sous l'effet des butées déviatrices, venir sensiblement s'appliquer contre la portion d'aiguille 2 extraite de la surface réceptrice 5R.

De manière remarquable :
- la pièce 9 mobile consiste en une pièce sensiblement plate, d'épaisseur e2 déterminée, comprenant:
   - deux faces opposées 9A, 9L dont une face 9A, dite d'appui, parce que destinée à prendre au moins localement appui contre la surface 5R réceptrice et, une face 9L, dite libre, destinée à recevoir une action d'application vers cette surface 5R réceptrice.
   - réalisée dans l'épaisseur e₂ de cette pièce 9, une découpe 10, d'une part, au bord de laquelle sont disposées les butées 9D déviatrices et, d'autre part, dont la section droite a une forme et une étendue au moins suffisante pour permettre l'engagement de chaque portion des lames 7 rapprochées l'une vers l'autre, après déviation par les butées 9D à cet effet,
- le support d'aiguille 3 de l'instrument présente, en retrait par rapport à sa face 3V ventrale, au moins une partie 31, 32, 33 dite première (31) qui, de section droite au moins équivalente à celle de l'ensemble que constituent les lames 7 lorsqu'elles sont au moins localement réunies contre l'aiguille 2, est destinée à coopérer de manière coulissante avec la découpe 10 pour
   - porter les lames 7 au niveau de la face 9A d'appui de la pièce 9 mobile, chacune avec leur face dorsale 7D localement en appui contre une butée 9D déviatrice de cette pièce 9 mobile et,
   - présenter au delà d'une face 3D du corps 3 qui, dite dorsale, est opposée à sa face ventrale 3V, au moins un moyen 11 de préhension de ce corps 3 et, au moins une butée 12B d'appui contre au moins une face 9L de la pièce 9 mobile qui est opposée à sa face 9A d'appui, de manière à entraver le dégagement de cette pièce 9,
- les lames 7 portent chacune au niveau de leur extrémité distale libre 7L, c'est-à-dire au niveau de leur extrémité 7L opposée à celle reliée au corps 3, des butées saillantes avant et arrière 7I,7J en vis-à-vis formées sur la surface 7D et destinées à coopérer avec des butées antagonistes 9A, 9M de la pièce 9 mobile pour constituer le moyen 8 de verrouillage (libérable) des lames 7 en situation d'appui contre l'aiguille 2 (position basse de l'élément mobile 9)..

La distance e₃ entre les butées avant et arrière 71, 7J est sensiblement égale à la distance E₂ - e₂ (E₂ : épaisseur totale du cadre 9, e₂ : épaisseur du passage central de ce cadre, entre les patins, ou épaisseur de ces patins).

A au moins certaines des butées 7I portées par les lames 7 en vue de l'immobilisation de la pièce 9 mobile, sont associées des rampes 7R destinées à coopérer avec la pièce 9 mobile en vue de déformer élastiquement et au moins localement, au moins l'un des éléments que sont ladite pièce 9 et les lames 7, de manière telle qu'après franchissement desdites rampes, le rappel élastique des parties déformées induise le verrouillage des butées antagonistes.

Par section droite de la découpe 10, on désigne sa section dans un plan orthogonal à son axe longitudinal, c'est-à-dire à l'axe fictif qu'elle comprend selon l'épaisseur de la pièce 9 mobile.

Par section droite du corps 3, on désigne sa section dans le même plan que celui de la section droite de la découpe 10.

De préférence, mais non limitativement, la face 9L destinée à coopérer avec la butée 12B s'opposant au dégagement du support d'aiguille 3 est la face 9L dite libre de la pièce 9 mobile.

De manière remarquable, la butée 12B s'opposant au dégagement du corps 3, est portée par ledit corps 3 de manière à être mobile entre deux positions dont, d'une part, une première position dans laquelle elle est effacée pour autoriser le montage du corps 3 dans la découpe 10 de la pièce 9 mobile et, d'autre part, une position seconde dans laquelle elle est opposée à la face 9L libre portée par la pièce 9 mobile, de manière à s'opposer au dégagement du corps 3.

De manière également remarquable, la butée 12B s'opposant au dégagement du support d'aiguille 3, est portée par un organe 12 qui, distinct de ce corps 3, est monté coulissant dans au moins une rainure 3R réservée dans ledit support 3, de manière à translater dans un plan approximativement orthogonal à l'aiguille 2 (axe 2L).

L'organe 12 est sensiblement plat et présente deux faces opposées 12B, 12D.

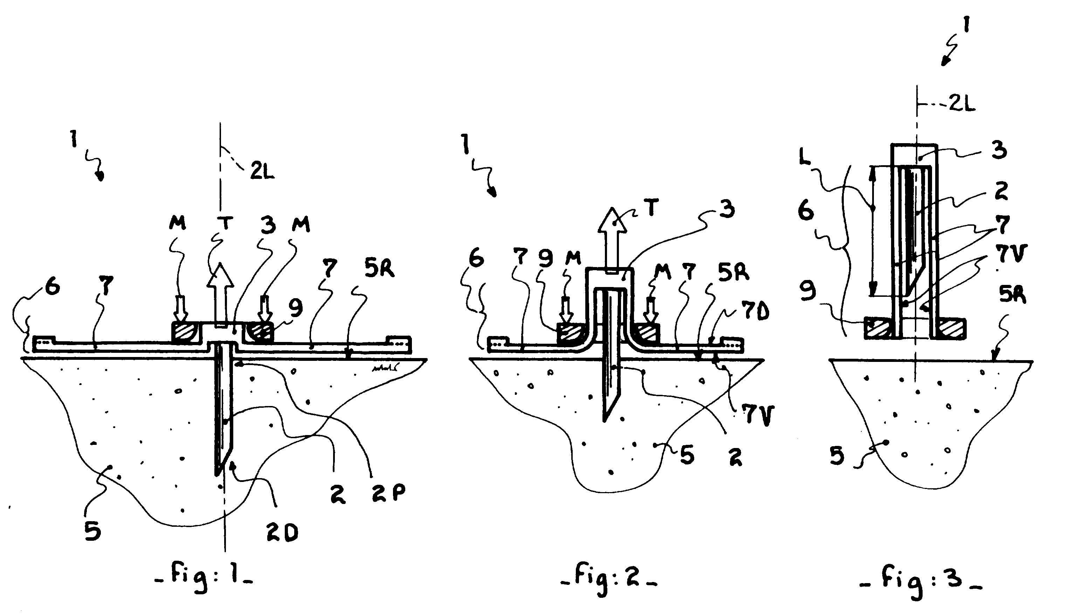

Les dimensions d'une telle plaque sont adaptées pour qu'elle interagisse en butée avec le cadre 9 lorsque celui-ci est en position haute, au dessus des languettes 7₁, 7₂ (figures 1 et 6).

Une fois montée sur le corps 3, la plaque de butée 12 est immobilisée par tout moyen à cet effet et, par exemple, au moyen de colle.

L'aiguille 2 est fixée sur le corps 3 par son extrémité proximale 2P et, à cette extrémité 2P, elle est coudée sensiblement à angle droit de manière à présenter un tronçon 2T de raccordement à une conduite 4 sensiblement parallèle à la surface 5R réceptrice.

De manière notable :
- le support d'aiguille 3 comprend, d'une part un perçage 3P axial destiné au passage de l'aiguille 2 entre sa face 3V, dite ventrale, et une face 3D opposée, dite dorsale, et d'autre part, réservée dans ladite face dorsale 3D, une portée 3L d'appui au moins indirect et de logement d'au moins une fraction du tronçon 2T de raccordement,
- l'organe 12 coulissant est constitué non seulement pour présenter la butée 12B entravant le déboîtement du corps 3 et de la pièce 9 mobile, mais également de manière à présenter une butée 12M de maintien du tronçon 2T de raccordement de l'aiguille contre la portée 3L du corps 3 qui l'accueille.

Egalement, l'organe 12 coulissant est conçu à la manière d'un capot qui coiffe le tronçon 2T de raccordement et la portée d'appui 3L.

De manière remarquable, le corps 3 comprend deux rainures 3R disposées pour permettre le guidage du coulissement de l'organe 12 avec sa butée 12M de maintien du tronçon 2T dans un plan parallèle à la face 3D dorsale dudit corps 3.

Avantageusement, le moyen 11 de préhension du corps 1A comprend :
- d'une part, distinct de l'instrument 1, une poignée amovible 13 de préhension digitale en vue de la manipulation de l'instrument pour autoriser au moins l'une des opérations que sont l'opération d'introduction ou l'opération d'extraction de l'aiguille hypodermique 2,
- d'autre part, solidaire du corps 3, au moins deux butées 3S, 3D, 12D disposées de manière à permettre la fixation amovible de l'organe 13 de préhension digitale.

Les butées 3S sont définies par des rebords latéraux 3', 3'' s'étendant au dessus de la rainure (des rainures) pour définir leur section supérieure.

L'organe 13 de préhension digitale consiste avantageusement en une pince.

De manière notable :
- les rainures 3R de guidage de l'organe 12 coulissant sont situées de part et d'autre d'un plan médian aux lames 6 et,
- les butées 3S, 3D, 12D destinées à la préhension du corps 3 sont situées au dessus desdites rainures 3R lorsqu'on observe l'instrument 1 avec l'aiguille orientée verticalement et vers le bas.

De manière notable, la poignée 13 consiste en une pince comprenant deux mors 13M et deux branches 13B d'actionnement, intégralement liées à l'un de ces mors 13M, le tout articulé autour d'une charnière intégrée 14.

En outre :
- les mors 13M présentent des surfaces disposées pour coopérer avec les butées 3S, 3D, 12D de préhension du corps 3 de l'instrument,
- les mors 13M et les branches 13B sont articulés de manière telle qu'une action de rapprochement des branches 13B induit une action d'écartement des mors 13M.

De manière notable:
- outre une première partie 31, d'une part, dont la section droite et de forme et d'étendue au moins suffisante pour permettre l'engagement de chaque portion des lames 7 placées en appui l'une contre l'autre, après déviation par les butées 9D déviatrices et, d'autre part, qui s'étend entre les faces 3V, 3D ventrale et dorsale du corps 3, le corps 3 comprend :
   - une seconde 32 et une troisième partie 33 qui, d'une part, s'étendent chacune au moins entre le plan des faces 3V ventrale et dorsale 3D du corps, font latéralement saillie de part et d'autre de la première partie 31 de manière à porter, au dessus du plan de la face 3D dite dorsale du corps 3 et de part de d'autre d'un plan médian aux lames 6, les butées 3S, 12S destinées à la préhension du corps 3,
- la découpe 10 de la pièce 9 mobile a une section droite ajustée à celle du corps 3 de manière à permettre le coulissement sur ledit corps 3.

Dans une forme remarquable de réalisation, d'une part, la découpe 10 de la pièce 9 mobile et le corps 3 qui porte l'aiguille 2 ont une section droite en forme de croix et, d'autre part, la pièce 9 mobile porte deux groupes de deux butées 9D déviatrices opposées et ces groupes sont disposés de manière telle que leurs butées coopèrent avec les faces 7D dorsales des lames 7 sensiblement au niveau des rives 7R qui les limitent latéralement.

## Revendications

1. Dispositif pour aiguille hypodermique comprenant:
- une aiguille (2, 2T) ayant une portion (2) adaptée pour être insérée dans un corps (5), ladite portion ayant une longueur suivant un axe (2L), ainsi qu'une extrémité distale effilée et une extrémité proximale (2P),
- un support d'aiguille (3) disposé autour d'un segment (2T) de l'aiguille vers l'extrémité proximale (2P) de celle-ci,
- un fourreau (6) de protection de ladite portion d'aiguille (2) comprenant au moins deux lames (7) plates d'une façon générale ayant des extrémités proximale et distale et réalisées en un matériau flexible,
- au moins deux zones d'articulation (71, 72), chaque zone d'articulation reliant l'extrémité proximale d'une lame (7) au support d'aiguille (3),
- un élément mobile (9) présentant à travers lui une ouverture axiale (10) au moins partiellement délimitée par des surfaces plates d'une façon générale (3₁, 3₂), de manière à être disposé autour des lames, le long de l'axe (2L) de ladite portion d'aiguille, entre des positions haute et basse, dans lesquelles, en position haute, l'élément mobile (9) est au-dessus des zones d'articulation (7₁, 7₂), et, en position basse, l'élément mobile est sensiblement à l'extrémité distale (7L) des lames et ses dites surfaces plates d'une façon générale (3₁, 3₂) coopèrent avec une surface extérieure (7D) des lames pour provoquer un mouvement de pivotement vers le bas desdites lames en les rapprochant de ladite portion d'aiguille (2) le long de son axe (2L), lorsque l'élément mobile est déplacé de sa position haute vers sa position basse.

2. Dispositif selon la revendication 1, caractérisé en ce que chaque lame (7) présente une surface intérieure (7V) opposée ladite surface extérieure (7D) et qui est dépourvue de toute gorge propre à contenir ladite portion d'aiguille (2), de sorte que dans la position basse de l'élément mobile (9), ladite portion d'aiguille demeure apparente entre les lames (7) et n'est pas enfermée à l'intérieur du fourreau de protection.

3. Dispositif selon la revendication 1 ou la revendication 2, caractérisé en ce que le segment d'aiguille autour duquel le support d'aiguille (3) est disposé est coudé.

4. Dispositif selon l'une quelconque des revendications 1 à 3, caractérisé en ce que l'élément mobile (9) comprend des butées déviatrices (7D) qui sont disposées sous lesdites surfaces plates d'une façon générale (3₁ ,32) de l'élément mobile et qui sont appliquées contre la surface extérieure (7D) des lames (7) pour coopérer avec elles lorsque l'élément mobile (9) est déplacé entre ses positions haute et basse.

5. Dispositif selon l'une quelconque des revendications précédentes, caractérisé en ce que l'élément mobile (9) se présente comme un cadre plat d'une façon générale suivant un plan général (9') sensiblement perpendiculaire à l'axe (2L) de ladite portion d'aiguille (2).

6. Dispositif selon l'une quelconque des revendications précédentes, caractérisé en ce que :
- les lames (7) présentent une largeur (I₁) et une épaisseur (e₁),
- l'élément mobile (9) présente deux patins latéraux (9₁, 9₂) s'étendant sensiblement perpendiculairement à l'axe (2L) de la portion d'aiguille et au plan général de chaque lame (7) alors que l'élément mobile (9) est dans sa position basse,
- la distance (I₂) entre les patins (9₁, 9₂) est supérieure à la largeur (I₁) des lames, et
- les patins (9₁, 9₂) présentent une épaisseur (e₂) qui est supérieure à l'épaisseur (e₁) des lames,
de sorte que lesdites lames (7) glissent entre les patins (9₁, 9₂) lorsque l'élément mobile (9) est déplacé entre ses positions haute et basse.

7. Dispositif selon l'une quelconque des revendications précédentes, caractérisé en ce que, sur leur surface extérieure (7D), les lames (7) présentent des butées saillantes avant (7J) et arrière (7i) disposées vers l'extrémité distale (7L) desdites lames, et ces butées (7i, 7J) présentent entre elles une distance (e₃) adaptée pour verrouiller de manière réversible entre elles l'élément mobile (9) dans la position basse de celui-ci.

8. Dispositif selon l'une quelconque des revendications précédentes, caractérisé en ce que :
- le support d'aiguille (3) présente au moins une rainure (3R) s'étendant perpendiculairement à l'axe de ladite portion d'aiguille (2),
- la rainure (3R) présente une longueur le long de son axe ainsi qu'une section supérieure longitudinalement délimitée par des rebords latéraux (3', 3"), et
- le dispositif comprend en outre une poignée amovible (13) ayant des extrémités proximale et distale, ladite extrémité distale de la poignée étant engagée dans la rainure, sous lesdits rebords latéraux (3', 3").

9. Dispositif selon la revendication 8, caractérisé en ce que la poignée amovible (13) présente deux mâchoires distales (13M) articulées d'une seule pièce avec deux branches de commande (13B), les mâchoires étant engagées dans ladite rainure (3R) du support d'aiguille (3) lorsque la poignée y est disposée.

10. Dispositif selon la revendication 8 ou la revendication 9, caractérisé en ce que le support d'aiguille (3) comprend en outre une plaque de butée (12) disposée à l'intérieur de ladite rainure (3R), sous la poignée amovible (13) lorsque celle-ci y est disposée, la plaque de butée (12) ayant des dimensions adaptées pour interagir en butée avec l'élément mobile (9) lorsque celui-ci est dans sa position haute.
